# EUROPEAN PATENT APPLICATION

(11) **EP 4 458 185 A1**
(43) Date of publication of application: **06.11.2024**
(21) Application number: 22923300.2
(22) Date of filing: 19.09.2022
(51) Int. Cl.: A24F 40/42, A24F 47/00, A61M 15/00, A61M 11/00

(54) **ANTI-COUNTERFEITING ELECTRONIC CIGARETTE HAVING LIMITED SERVICE LIFE, AND CONTROL METHOD THEREFOR**

(30) Priority: 27.01.2022 CN 202210111959
(71) Applicant: Huizhou Happy Vaping Technology Limited, Huizhou, Guangdong 516000 (CN)
(72) Inventor: LIN, Guangrong, Shenzhen, Guangdong 518104 (CN); ZHENG, Xianbin, Shenzhen, Guangdong 518104 (CN)
(74) Representative: Hannke, Christian
(86) International application number: PCT/CN2022/119497
(87) International publication number: WO 2023/142496

(57) **Abstract**

An anti-counterfeiting electronic cigarette having a limitation of service life and a control method thereof are provided. The electronic cigarette comprises a liquid storage part (1), a vaporizer (2), and a battery part (3), which are sequentially detachably connected; The liquid storage part (1) is provided with an NFC electronic tag and a first information storage, the NFC electronic tag is set with a dynamic anti-counterfeiting code, and the first information storage is configured to store data including a first cigarette liquid consumption simulation count value; The vaporizer (2) is provided with an ID encryption unit and a second information storage, the ID encryption unit is set with an identification code, and the second information storage is configured to store data including a second cigarette liquid consumption simulation count value; The battery part (3) is provided with a microcontroller, an airflow sensor, and an NFC antenna, the microcontroller is provided with an NFC identification module, an NFC read/write control module, an ID encryption unit communication module, an ID encryption unit identification module, and an ID encryption unit read/write control module. It has advantages that the vaporizer which is reusable can avoid waste. It avoids a damage caused by dry-heating, limits the service life of the vaporizer, and avoids the use of counterfeit products.

## Description

### TECHNICAL FIELD

The disclosure relates to the field of the electronic cigarette preheating technology, and more particularly, the disclosure relates to an anti-counterfeiting electronic cigarette having a limitation of service life and a control method thereof.

### BACKGROUND

Usually, an electronic vaporizing device, e.g. an electronic cigarette, heats and vaporizes the liquid substance such as the cigarette liquid, the medicine liquid and the herbal essence liquid, to produce aerosol or vapor for users to vape. Different from tobacco cigarettes which may be directly combusted to produce smoke, the electronic cigarette produces vapor without cigarette tar and the harmful particles. Thus, the electronic cigarettes are widely used.

Generally, an electronic vaporizing device or an electronic cigarette in the market includes a battery part and a vaporizer part which are connected with each other. The battery part supplies power for the vaporizer part and provides control for it. The electronic cigarette liquid is usually stored in the vaporizing chamber provided on the vaporizer part. In order to ensure authentic guarantee and protect consumers' rights and interests, users are usually not allowed to refill the vaporizing liquid or the cigarette liquid by themselves, and the vaporizer part has to be discarded when the vaporizing liquid or the electronic cigarette liquid runs out. With the continuous development of electronic cigarettes and the ever-increasing demands of people on the quality of electronic cigarettes, the vaporizer part uses vaporizing components such as ceramic vaporizing cores and precious metal heating components which are relatively expensive. In such a case, if the vaporizer part is discarded when the cigarette liquid runs out, it will result in great waste of resources. The electronic cigarette products in the market are in the face of some issues and technical difficulties to be addressed, that is, how to provide a separable arrangement of the liquid storage component from the vaporizing component to save resources, a consequent anti-counterfeit control, a reuse of vaporizing components, and a control of service life under quality assurance.

### SUMMARY

### Technical problems

The disclosure aims to provide an anti-counterfeiting electronic cigarette having a limitation of service life and a control method thereof, to overcome the technical issues.

### Solutions for the problems

### Technical solutions

A technical solution of the disclosure is provided as follows. An anti-counterfeiting electronic cigarette having a limitation of service life, comprising a liquid storage part, a vaporizer, and a battery part, which are sequentially detachably connected;
The liquid storage part is provided with an NFC electronic tag and a first information storage, the NFC electronic tag is set with a refreshable dynamic anti-counterfeiting code, and the first information storage is configured to store data including a first cigarette liquid consumption simulation count value;
The vaporizer is provided with an ID encryption unit and a second information storage, the ID encryption unit is set with an identification code, and the second information storage is configured to store data including a second cigarette liquid consumption simulation count value;
The battery part is provided with a microcontroller, an airflow sensor, and an NFC antenna, wherein the microcontroller is provided with an NFC identification module, an NFC read/write control module, an ID encryption unit communication module, an ID encryption unit identification module, and an ID encryption unit read/write control module.

Preferably, the first information storage may be disposed on the NFC electronic tag, and the second information storage may be disposed in the ID encryption unit.

Preferably, the microcontroller may be further provided with a third information storage.

Preferably, the microcontroller may be further provided with a puff counter or a power consumption counter, and the first cigarette liquid consumption simulation count value or the second cigarette liquid consumption simulation count value may be derived from a count made by the puff counter or power consumption counter.

Preferably, the microcontroller may be further provided with a liquid storage part replacement counter, and the second information storage may be configured to store data including a cumulative value of liquid storage part replacement times.

Preferably, the microcontroller may be further provided with a timer for cumulatively calculating a vaping duration and a standby duration of the electronic cigarette.

Preferably, the battery part may be further provided with a reminder unit.

A further technical solution of the disclosure is provided as follows. A control method of an anti-counterfeiting electronic cigarette having a limitation of service life, comprising steps of:
(1) Presetting related parameters of the microcontroller and initializing data;
(2) Activating the microcontroller to work once the vaporizer is connected to the battery part;
(3) By the microcontroller, reading the identification code in the ID encryption unit of the vaporizer by means of the encryption unit communication module and the ID encryption unit read/write control module, and determining whether the identification code is correct by means of the ID encryption unit identification module; if not, go to the next step, if yes, go to the step (5);
(4) By means of the reminder unit, prompting to replace the vaporizer with a genuine vaporizer, and after a few seconds, going to the step (18);
(5) Generating a new identification code by means of the microcontroller, and writing it into the ID encryption unit by means of the ID encryption unit read/write control module;
(6) By the microcontroller, reading the second cigarette liquid consumption simulation count value stored in the second information storage by means of the ID encryption unit communication module and the ID encryption unit read/write control module, and determining whether the value exceeds a second set threshold; if yes, go to the next step, if not, go to the step (8);
(7) By means of the reminder unit, prompting that the vaporizer has been used up to reach the service life and prompting to replace a new vaporizer, and after a few seconds, going to the step (18);
(8) By the microcontroller, reading the dynamic anti-counterfeiting code in the NFC electronic tag of the liquid storage part by means of the NFC antenna and the NFC read/write control module, and determining whether the dynamic anti-counterfeiting code is accurate by means of the NFC identification module; if not, go to the next step, if yes, go to the step (10);
(9) By means of the reminder unit, prompting to replace the liquid storage part with a genuine liquid storage part, and after a few seconds, going to the step (18);
(10) Generating a new dynamic anti-counterfeiting code by means of the microcontroller, and writing it into the NFC electronic tag by means of the NFC read/write control module;
(11) By the microcontroller, reading the first cigarette liquid consumption simulation count value stored in the first information storage by means of the NFC antenna and the NFC read/write control module, and determining whether the value exceeds a first set threshold; if yes, go to the next step, if not, go to the step (13);
(12) By means of the reminder unit, prompting that the liquid storage part has been used up to reach the service life and prompting to replace a new liquid storage part, and after a few seconds, going to the step (18);
(13) Entering a standby state of the whole device, and cumulatively calculating a standby time by means of the timer;
(14) By means of the airflow sensor, detecting whether there is an air flow of vaping; if yes, go to the next step, if not, go to the step (17);
(15) Performing cumulative counting by means of the puff counter or the power consumption counter;
(16) By means of the microcontroller, converting the cumulative count values and sending them to the first information storage and the second information storage, to update the first cigarette liquid consumption simulation count value and the second cigarette liquid consumption simulation count value;
(17) Determining whether the standby time exceeds a preset time by means of the microcontroller; if yes, go to the next step, if not, go back to the step (13);
(18) Shutting down the entire device.

### Advantages of the disclosure

### Advantages

The disclosure provides the electronic cigarette which has a structure consisting of three parts, i.e., a battery part, a vaporizer, and a liquid storage part. When the vaporizing liquid or the cigarette liquid stored in the liquid storage part runs out, the liquid storage part may be discarded and the vaporizer may be assembled with a new liquid storage part for use, thereby avoiding waste of resources. When the cigarette liquid stored in the liquid storage part runs out, it should determine that the cigarette liquid runs out and automatically shut down depending on cigarette liquid consumption simulation count values, thereby avoiding a damage of the vaporizer caused by dry-heating when the cigarette liquid runs out and preventing the produced vapor from getting a burning taste. Besides, the disclosure determines the cigarette liquid consumption of the vaporizer depending on the cigarette liquid consumption simulation count values, and limits the number of uses or service life of the vaporizer to guarantee the consistent quality of the vaporizer. The disclosure, which comprises the liquid storage part set with the dynamic anti-counterfeiting code and the vaporizer set with the identification code, avoids the use of counterfeit products and prevents the harm of counterfeit products to consumers.

### BRIEF DESCRIPTION OF THE DRAWINGS

### Description of the drawings

FIG.1 is a schematic structural view illustrating an anti-counterfeiting electronic cigarette having a limitation of service life according to the disclosure in a disassembled state;
FIG.2 is a schematic view illustrating functional modules of an anti-counterfeiting electronic cigarette having a limitation of service life according to the disclosure;
FIG.3 is a flow chart illustrating a control method of an anti-counterfeiting electronic cigarette having a limitation of service life.

### DETAILED DESCRIPTION OF ILLUSTRATED EMBODIMENTS

### Preferred embodiments of the disclosure

To avoid the great waste of resources caused by discard of the vaporizer when the cigarette liquid runs out, the disclosure provides an electronic cigarette having a structure consisting of three parts, including a battery part, a vaporizer, and a liquid storage part. In other words, the structure of a conventional vaporizer part is designed to consist of two parts, i.e., the vaporizer and the liquid storage part, which are detachably connected. When the vaporizing liquid or the cigarette liquid stored in the liquid storage part runs out, the liquid storage part may be discarded and the vaporizer may be assembled with a new liquid storage part for use, thereby avoiding waste. When the cigarette liquid stored in the liquid storage part runs out, it should automatically determine that the cigarette liquid runs out and automatically shut down, thereby avoiding a damage of the vaporizer caused by dry-heating when the cigarette liquid runs out and preventing the produced vapor from getting a burning taste. Besides, the vaporizer which has a certain service life cannot be used indefinitely, and the consistent quality of the vaporizer can be guaranteed only during the normal service life. Therefore, it is necessary to limit the number of uses or service life. In the case that the electronic cigarette has the structure consisting of these three parts, counterfeit vaporizers with a low cost may appear in the market. In order to prevent the harm of counterfeit products to consumers, it is necessary to conduct identity verification and comparison between the liquid storage part with the vaporizer and the battery part. If a non-genuine product is identified, it should shut down.

### Embodiments

### Embodiments of the disclosure

The disclosure will be further explained in detail with reference to the drawings.

### Embodiment 1

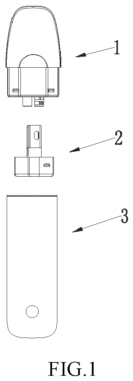

Referring to FIG.1, an anti-counterfeiting electronic cigarette having a limitation of service life of the disclosure comprises a liquid storage part 1, a vaporizer 2, and a battery part 3, which are sequentially detachably connected. The liquid storage part 1, which may be referred to as a cartridge, is dedicated to store the vaporizing liquid or the cigarette liquid. The cartridge has few accessories and thus a low cost. The vaporizer 2 is configured to connect the liquid storage part 1 and heat and vaporize the vaporizing liquid or the cigarette liquid stored in the liquid storage part 1. The battery part 3 is provided with a battery for supplying power to the vaporizer 2. When the cigarette liquid stored in the liquid storage part 1 runs out, the vaporizer 2 may be separated from the liquid storage part 1 and assembled with a new liquid storage part 1, to allow for the reuse of the vaporizer 2.

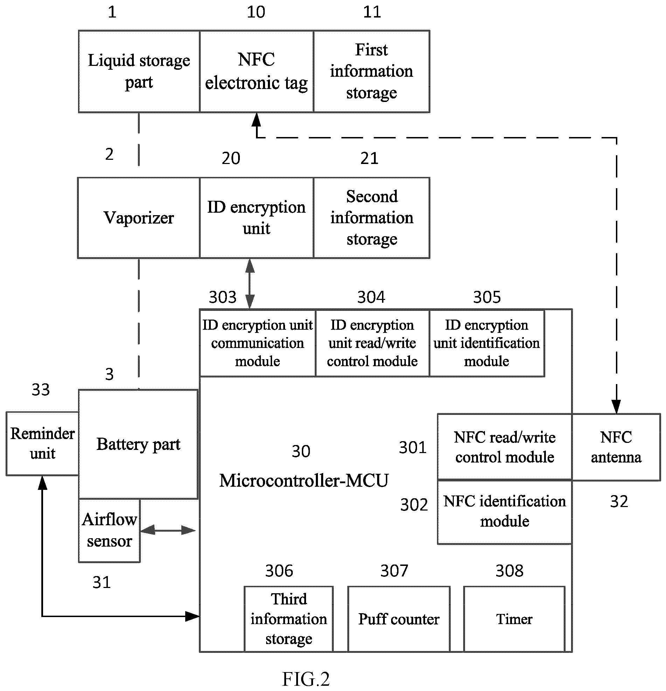

Referring to FIG.2, the liquid storage part 1 is arranged with an NFC electronic tag 10 and a first information storage 11, wherein the NFC electronic tag 10 is provided with a refreshable dynamic anti-counterfeiting code, and the first information storage 11 is configured to store data including a first cigarette liquid consumption simulation count value.

The vaporizer 2 is arranged with an ID encryption unit 20 and a second information storage 21, wherein the ID encryption unit 20 is provided with an identification code, and the second information storage 21 is configured to store data including a second cigarette liquid consumption simulation count value.

The battery part 3 is arranged with a microcontroller 30, an airflow sensor 31, an NFC antenna 32, and a reminder unit 33, wherein the microcontroller 30 comprises an NFC read/write control module 301, an NFC identification module 302, an ID encryption unit communication module 303, an ID encryption unit read/write control module 304, and an ID encryption unit identification module 305.

The microcontroller 30 is further provided with a third information storage 306, a puff counter or power consumption counter 307, a timer 308, wherein the third information storage 306 is configured to store an identification code, a dynamic anti-counterfeiting code, and the like, and the timer 308 is configured to cumulatively calculate the vaping duration and standby duration of the electronic cigarette. The first cigarette liquid consumption simulation count value and the second cigarette liquid consumption simulation count value are derived from the count made by the puff counter or power consumption counter 307.

In further embodiments, the first information storage 11 may be arranged on the NFC electronic tag 10, and the second information storage 21 may be arranged in the ID encryption unit 20.

In the present embodiment, once the vaporizer 2 is connected to the battery part 3, the microcontroller 30 may be activated to work; The microcontroller 30 reads the identification code of the ID encryption unit 20 provided on the vaporizer 2 by means of the ID encryption unit communication module 303 and the ID encryption unit read/write control module 304, and identifies and determines whether the identification code is correct by means of the ID encryption unit identification module 305, if not, the reminder unit 33 prompts to replace the vaporizer with a genuine vaporizer, and then shutdown protection is triggered.

When the vaporizer 2 is connected to the battery part and the liquid storage part 1 is connected to the vaporizer 2 too, the microcontroller 30 reads the dynamic anti-counterfeiting code of the NFC electronic tag 10 provided on the liquid storage part 1 by means of the NFC antenna 32 and the NFC read/write control module 301, and identifies and determines whether the dynamic anti-counterfeiting code is accurate by means of the NFC identification module 302, if not, the reminder unit 33 prompts to replace the liquid storage part with a genuine liquid storage part, and then trigger shutdown protection. The NFC antenna 32 and the NFC electronic tag 10 communicate via wireless signals.

When the entire electronic cigarette enters the standby state, it may be vaped by a user. The airflow sensor 31 detects whether there is an air flow produced by vaping. When an air flow is detected, the puff counter 307 records one puff. The microcontroller 30 calculates the cigarette liquid consumption simulation count values from the number of puffs and sends them to the first information storage 11 of the liquid storage part 1 and the second information storage 21 of the vaporizer 2, respectively.

When using the puff counter 307, the simulation conversion of the cigarette liquid consumption may have a deviation due to different puff amounts and durations per single puff of different person. In further embodiments, the puff counter may be replaced by the power consumption counter. The power consumption counter which simulates the cigarette liquid consumption by calculating power consumption during vaping of the user approximates actual consumption.

The liquid storage part 1 is provided with the first information storage 11 which stores the first cigarette liquid consumption simulation count value indicating an accumulated cigarette liquid consumption of the liquid storage part. Even when the liquid storage part is removed during use, the first cigarette liquid consumption simulation count value will not be zeroed. Once the liquid storage part is mounted again for use, the first cigarette liquid consumption simulation count value continues to accumulate. During use, the microcontroller 30 reads and writes the first cigarette liquid consumption simulation count value stored in the first information storage 11 by the NFC antenna 32 and the NFC read/write control module 301, and perform comparison to determine whether the value exceeds a first set threshold. For example, the number of puffs may be set as 300, when it is determined that the value exceeds the first set threshold 300, it indicates that the cigarette liquid of the liquid storage part has run out, the reminder unit 33 prompts to replace a new liquid storage part, and the shutdown protection is triggered.

The vaporizer 2 is provided with the second information storage 21 which stores the second cigarette liquid consumption simulation count value indicating an accumulated cigarette liquid consumption of the vaporizer. Even when the vaporizer is removed during use, the second cigarette liquid consumption simulation count value will not be zeroed. Once the vaporizer is mounted again for use, the second cigarette liquid consumption simulation count value continues to accumulate. During use, the microcontroller 30 reads and writes the second cigarette liquid consumption simulation count value stored in the second information storage 21 by the ID encryption unit communication module 303 and the ID encryption unit read/write control module 304, and perform comparison to determine whether the value exceeds a second set threshold. For example, the number of puffs may be set as 1500, when it is determined that the value exceeds the second set threshold 1500, it indicates that the cumulative amount of the cigarette liquid vaporized by the vaporizer has reached the limit, i.e., the vaporizer 2 has been used up to reach the designed service life. The reminder unit 33 prompts to replace a new vaporizer, and the shutdown protection is triggered.

In further embodiments on such basis, the microcontroller 30 may be further provided with a liquid storage part replacement counter (not shown in the drawings). The second information storage 21 stores data including the cumulative value of the liquid storage part replacement times. When the first cigarette liquid consumption simulation count value stored in the first information storage 11 exceeds the first set threshold, for example exceeds the set number of puffs 300, it indicates that the cigarette liquid of the liquid storage part has run out and a replacement is required. At this moment, the liquid storage part replacement counter performs counting and calculates the cumulative value of the liquid storage part replacement times of the second information storage 21. When the cumulative value of the liquid storage part replacement times reaches a set replacement times, for example the liquid storage part 1 has been replaced for 5 times, it indicates that the vaporizer 2 has been used up to reach the designed service life. The reminder unit 33 prompts to replace a new vaporizer, and the shutdown protection is triggered.

### Embodiment 2

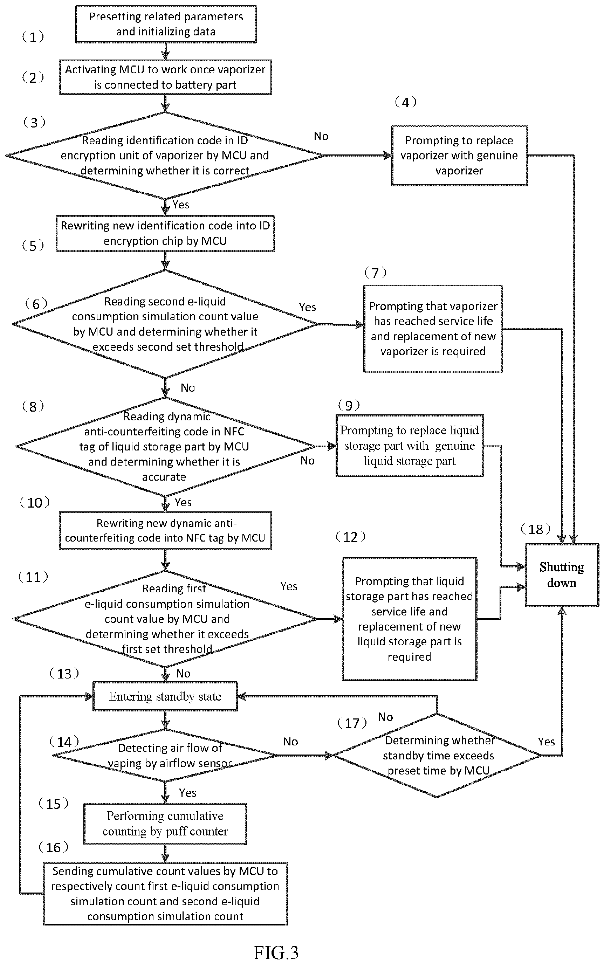

Referring to FIGs.1-3, a control method of an anti-counterfeiting electronic cigarette having a limitation of service life of the disclosure comprises steps as follows.
(1) Presetting related parameters of the microcontroller 30 and initializing data;
(2) Activating the microcontroller 30 to work when the vaporizer 2 is connected to the battery part 3;
(3) By using the microcontroller (MCU) 30, reading the identification code in the ID encryption unit of the vaporizer by means of the encryption unit communication module 303 and the ID encryption unit read/write control module 304, and determining whether the identification code is correct by means of the ID encryption unit identification module 305; if not, go to the next step, if yes, go to the step (5);
(4) By means of the reminder unit 33, prompting to replace the vaporizer 2 with a genuine vaporizer, and after a few seconds, going to the step (18);
(5) Generating a new identification code by means of the microcontroller 30, and writing the new identification code into the ID encryption unit by means of the ID encryption unit read/write control module 304;
(6) By using the microcontroller 30, reading the second cigarette liquid consumption simulation count value stored in the second information storage 21 by means of the ID encryption unit communication module 303 and the ID encryption unit read/write control module 304, and determining whether the value exceeds the second set threshold; if yes, go to the next step, if not, go to the step (8);
(7) By means of the reminder unit 33, prompting that the vaporizer 2 has been used up to reach the service life and prompting to replace a new vaporizer, and after a few seconds, going to the step (18);
(8) By using the microcontroller 30, reading the dynamic anti-counterfeiting code in the NFC electronic tag 10 of the liquid storage part 1 by means of the NFC antenna 32 and the NFC read/write control module 301, and determining whether the dynamic anti-counterfeiting code is accurate by means of the NFC identification module 302; if not, go to the next step, if yes, go to the step (10);
(9) By means of the reminder unit 33, prompting to replace the liquid storage part 1 with a genuine liquid storage part, and after a few seconds, going to the step (18);
(10) Generating a new dynamic anti-counterfeiting code by means of the microcontroller 30, and writing the new dynamic anti-counterfeiting code into the NFC electronic tag 10 by means of the NFC read/write control module 301;
(11) By using the microcontroller 30, reading the first cigarette liquid consumption simulation count value stored in the first information storage 11 by means of the NFC antenna 32 and the NFC read/write control module 301, and determining whether the value exceeds the first set threshold; if yes, go to the next step, if not, go to the step (13);
(12) By means of the reminder unit 33, prompting that the liquid storage part 1 has been used up to reach the service life and prompting to replace a new liquid storage part, and after a few seconds, going to the step (18);
(13) Entering a standby state of the whole device, and calculating the total standby time by means of the timer 308;
(14) By means of the airflow sensor 31, detecting whether there is an air flow produced by vaping; if yes, go to the next step, if not, go to the step (17);
(15) By means of the puff counter 307, performing cumulative counting;
(16) Converting the cumulative amounts by means of the microcontroller 30, and sending them to the first information storage 11 and the second information storage 21, to update the first cigarette liquid consumption simulation count value and the second cigarette liquid consumption simulation count value;
(17) Determining whether the standby time exceeds the preset time by means of the microcontroller 30; if yes, go to the next step, if not, go back to the step (13);
(18) Shutting down the entire electronic cigarette device.

In further embodiments, the puff counter in the above steps may be replaced by the power consumption counter.

### Industrial applicability

All the above are merely preferred embodiments of the disclosure. The present invention is intended to cover all equivalent arrangements and modifications derived from the claims of the present invention.

## Claims

1. An anti-counterfeiting electronic cigarette having a limitation of service life, comprising a liquid storage part (1), a vaporizer (2), and a battery part (3), which are sequentially detachably connected;
wherein the liquid storage part (1) is provided with an NFC electronic tag (10) and a first information storage (11), the NFC electronic tag (10) is set with a refreshable dynamic anti-counterfeiting code, and the first information storage (11) is configured to store data including a first cigarette liquid consumption simulation count value;
wherein the vaporizer (2) is provided with an ID encryption unit (20) and a second information storage (21), the ID encryption unit (20) is set with an identification code, and the second information storage (21) is configured to store data including a second cigarette liquid consumption simulation count value; and
wherein the battery part (3) is provided with a microcontroller (30), an airflow sensor (31), and an NFC antenna (32), the microcontroller (30) is provided with an NFC identification module (302), an NFC read/write control module (301), an ID encryption unit communication module (303), an ID encryption unit identification module (304), and an ID encryption unit read/write control module (305).

2. The anti-counterfeiting electronic cigarette having the limitation of service life according to claim 1, wherein the first information storage (11) is disposed on the NFC electronic tag (10), and the second information storage (21) is disposed in the ID encryption unit (20).

3. The anti-counterfeiting electronic cigarette having the limitation of service life according to claim 1, wherein the microcontroller (30) is further provided with a third information storage (306).

4. The anti-counterfeiting electronic cigarette having the limitation of service life according to claim 1, wherein the microcontroller (30) is further provided with a puff counter or a power consumption counter (307), and the first cigarette liquid consumption simulation count value or the second cigarette liquid consumption simulation count value are derived from a count made by the puff counter or power consumption counter (307).

5. The anti-counterfeiting electronic cigarette having the limitation of service life according to claim 4, wherein the microcontroller (30) is further provided with a liquid storage part replacement counter, and the second information storage is configured to store data including a cumulative value of liquid storage part replacement times.

6. The anti-counterfeiting electronic cigarette having the limitation of service life according to claim 1, wherein the microcontroller (30) is further provided with a timer (308) for cumulatively calculating a vaping duration and a standby duration of the electronic cigarette.

7. The anti-counterfeiting electronic cigarette having the limitation of service life according to claim 1, wherein the battery part is further provided with a reminder unit (33).

8. A control method of an anti-counterfeiting electronic cigarette having a limitation of service life applied to the anti-counterfeiting electronic cigarette having the limitation of service life according to claim 1, comprising steps of:
(1) presetting related parameters of the microcontroller and initializing data;
(2) activating the microcontroller to work once the vaporizer is connected to the battery part;
(3) by the microcontroller, reading the identification code in the ID encryption unit of the vaporizer by means of the encryption unit communication module and the ID encryption unit read/write control module, and determining whether the identification code is correct by means of the ID encryption unit identification module; if not, go to the next step, if yes, go to the step (5);
(4) by means of the reminder unit, prompting to replace the vaporizer with a genuine vaporizer, and after a few seconds, going to the step (18);
(5) generating a new identification code by means of the microcontroller, and writing the new identification code into the ID encryption unit by means of the ID encryption unit read/write control module;
(6) by the microcontroller, reading the second cigarette liquid consumption simulation count value stored in the second information storage by means of the ID encryption unit communication module and the ID encryption unit read/write control module, and determining whether the value exceeds a second set threshold; if yes, go to the next step, if not, go to the step (8);
(7) by means of the reminder unit, prompting that the vaporizer has been used up to reach the service life and prompting to replace a new vaporizer, and after a few seconds, going to the step (18);
(8) by the microcontroller, reading the dynamic anti-counterfeiting code in the NFC electronic tag of the liquid storage part by means of the NFC antenna and the NFC read/write control module, and determining whether the dynamic anti-counterfeiting code is accurate by means of the NFC identification module; if not, go to the next step, if yes, go to the step (10);
(9) by means of the reminder unit, prompting to replace the liquid storage part with a genuine liquid storage part, and after a few seconds, going to the step (18);
(10) generating a new dynamic anti-counterfeiting code by means of the microcontroller, and writing the new dynamic anti-counterfeiting code into the NFC electronic tag by means of the NFC read/write control module;
(11) by the microcontroller, reading the first cigarette liquid consumption simulation count value stored in the first information storage by means of the NFC antenna and the NFC read/write control module, and determining whether the value exceeds a first set threshold; if yes, go to the next step, if not, go to the step (13);
(12) by means of the reminder unit, prompting that the liquid storage part has been used up to reach the service life and prompting to replace a new liquid storage part, and after a few seconds, going to the step (18);
(13) entering a standby state of the whole device, and cumulatively calculating a standby time by means of the timer;
(14) by means of the airflow sensor, detecting whether there is an air flow of vaping; if yes, go to the next step, if not, go to the step (17);
(15) performing cumulative counting by means of the puff counter or the power consumption counter;
(16) by means of the microcontroller, converting the cumulative counts and sending them to the first information storage and the second information storage, to update the first cigarette liquid consumption simulation count value and the second cigarette liquid consumption simulation count value;
(17) determining whether the standby time exceeds a preset time by means of the microcontroller; if yes, go to the next step, if not, go back to the step (13); and
(18) shutting down the entire device.
